# EUROPEAN PATENT SPECIFICATION

(11) **EP 2 648 709 B1**
(45) Date of publication and mention of the grant of the patent: **24.07.2019**
(21) Application number: 11808284.1
(22) Date of filing: 08.12.2011
(51) Int. Cl.: A61K 31/145, A61K 9/127, A61K 9/51, A61P 35/00

(54) **DISULFIRAM FORMULATION AND USES THEREOF**
DISULFIRAM-FORMULIERUNG UND VERWENDUNGEN DAVON
FORMULATION DE DISULFIRAM ET UTILISATIONS DE CELLE-CI

(30) Priority: 09.12.2010 GB 201020860
(43) Date of publication of application: 16.10.2013
(73) Proprietor: Disulfican Limited, Pendeford Business Park Wolverhampton, WV9 5HD (GB)
(72) Inventor: WANG, Weiguang, Wolverhampton West Midlands WV1 1LY (GB)
(74) Representative: Barker Brettell LLP
(86) International application number: PCT/GB2011/052439
(87) International publication number: WO 2012/076897

(56) References cited:
- WO-A1-01/17522
- WO-A2-2005/009338
- WO-A2-2008/068746
- YOSHIMASA ITO ET AL: "Preventive Effect of Eye Drops of Liposomes Containing Disulfiram and Cefmetazole on Selenite-Induced Cataract in Rat Pups", JOURNAL OF OLEO SCIENCE, vol. 55, no. 1, 1 January 2006 (2006-01-01), pages 15-22, XP055018122, ISSN: 1345-8957, DOI: 10.5650/jos.55.15 [retrieved on 2012-02-01]
- Löbler M et al: "Drug delivery by nanoparticles - facind the obstacles", IFMBE Proceedings , vol. 22 2008, pages 2335-2338, XP002668497, Retrieved from the Internet: URL:https://springerlink3.metapress.com/co ntent/m12257v543873q13/resource-secured/?t arget=fulltext.pdf&sid=uspbgebvtqgdplrjaxw 3tpso&sh=www.springerlink.com [retrieved on 2012-02-01]
- Wu Dao Cheng: "Diethyldithiocarbamate may enhance the antitumor effect of adriamycin nanoparticle-lipiodol emulsion", Chinese Journal of Cancer Research, vol. 14, no. 3 2002, pages 192-194, XP002668498, Retrieved from the Internet: URL:http://www.springerlink.com/content/h6 616pn8481u1724/fulltext.pdf [retrieved on 2012-02-01]
- ITO YOSHIMASA ET AL: "Effects of lipid composition on the transcorneal penetration of liposomes containing disulfiram, a potential anti-cataract agent, in the rabbit", BIOLOGICAL & PHARMACEUTICAL BULLETIN (OF JAPAN), PHARMACEUTICAL SOCIETY OF JAPAN, TOKYO, JP, vol. 23, no. 3, 1 March 2000 (2000-03-01), pages 327-333, XP003032369, ISSN: 0918-6158
- RAJENDER REDDY K: "Controlled-Release, Pegylation Liposomal Formulations: New Mecanisms in the Delivery of Injectable Drugs", ANNALS OF PHARMACOTHERAPY, HARVEY WHITNEY BOOKS COMPANY, US, vol. 34, 1 July 2000 (2000-07-01), pages 915-923, XP003032370, ISSN: 1060-0280
- DRUMMOND D C ET AL: "OPTIMIZING LIPOSOMES FOR DELIVERY OF CHEMOTHERAPEUTIC AGENTS TO SOLID TUMORS", PHARMACOLOGICAL REVIEWS, AMERICAN SOCIETY FOR PHARMACOLOGY AND EXPERIMENTAL THERAPEUTICS, US, vol. 51, no. 4, 1 January 1999 (1999-01-01), pages 691-743, XP009059957, ISSN: 0031-6997
- JOHANSSON B: "A review of the pharmacokinetics and pharmacodynamics of disulfiram and its metabolites.", ACTA PSYCHIATRICA SCANDINAVICA. SUPPLEMENTUM 1992, vol. 369, 1992, pages 15-26, ISSN: 0065-1591
- SILVERMAN JEFFREY A ET AL: "Marqibo (R) (vincristine sulfate liposome injection) improves the pharmacokinetics and pharmacodynamics of vincristine", CANCER CHEMOTHERAPY AND PHARMACOLOGY, vol. 71, no. 3, March 2013 (2013-03), pages 555-564, ISSN: 0344-5704(print)
- ENEANYA D I ET AL: "The actions of metabolic fate of disulfiram.", ANNUAL REVIEW OF PHARMACOLOGY AND TOXICOLOGY 1981, vol. 21, 1981, pages 575-596, ISSN: 0362-1642

## Description

The invention relates to a novel disulfiram, or derivative thereof, formulation and uses thereof, in particular uses for the treatment of cancer.

Disulfiram has been used for over 50 years for the treatment of alcohol abuse. Disulfiram produces sensitivity to alcohol which results in a highly unpleasant reaction when a patient under treatment ingests even small amounts of alcohol. Disulfiram blocks the oxidation of alcohol at the acetaldehyde stage. After disulfiram intake the concentration of acetaldehyde occurring in the blood may be 5 to 10 times higher than that found during metabolism of the same amount of alcohol in a subject who has not ingested disulfiram. The accumulation of the acetaldehyde in the blood produces a complex of highly unpleasant symptoms referred to as the disulfiram-alcohol reaction. This reaction, which is proportional to the dosage of both disulfiram and alcohol, will persist as long as alcohol is being metabolized. Disulfiram plus even small amounts of alcohol produces flushing, throbbing in the head and neck, throbbing headache, respiratory difficulty, nausea, copious vomiting, sweating, thirst, chest pain, palpitation, hyperventilation, tachycardia, hypotension, syncope, marked uneasiness, weakness, vertigo, blurred vision and confusion.

Disulfiram has been demonstrated to be highly toxic to several different kinds of cancer cell lines in vitro (Wang W et al (2003) Int J Cancer 104(4):504-11; Cen De et al (2004) J Med Chem 47(27):6914-2). The effective cytotoxic concentration of disulfiram observed in cancer cell lines is at nanomolar levels, this is significantly more efficient than many clinically used anticancer drugs. Despite its very encouraging anticancer effect *in vitro*, there are very few reports demonstrating the anticancer activity of disulfiram in animal studies (Iljin K et al Clin Cancer Res 2009;15(19):6070-8; Brar S S et al Mol Cancer Ther 2004;3(9):1049-60; Chen D et al Cancer Res 2006;66(21):10425-33). Furthermore, such studies demonstrate that disulfiram must be administered at extremely high doses in mice to be effective, such doses would not be tolerable in human cancer patients. There have been several completed and on-going clinical trials using disulfiram for cancer therapeutics (Verma S et al. Am J Clin Oncol 1990; 13(2): 119-24) but no positive outcomes have been reported. The discrepancy between *in vitro*, *in vivo* and in clinic data has resulted in the conclusion that disulfiram is not a suitable therapy for the treatment of cancer in humans.

Thus, until now, it has not been possible to use disulfiram as an anticancer agent *in vivo* in humans. The potential clinical application of disulfiram in cancer treatment is hampered by the limitations of the currently available disulfiram oral formulation. After oral administration, disulfiram is extremely unstable in acidic gastric juice and most of the orally administered disulfiram is rapidly decomposed into carbon disulphide (CS2) and diethylamine (DEA) (Johansson B. A Acta Psychiatr Scand Suppl 1992;369:15-26). The absorbed disulfiram is enriched in the liver and promptly degraded in the blood stream by glutathione reductase system in erythrocytes (half-life: 4 minutes). Therefore, the blood concentration of disulfiram after oral administration of a 500 mg dose is below the limit of detection. This does not affect its antialcoholism effect because the orally administered disulfiram is quickly delivered into the liver where it inhibits ALDH. The prompt degradation of disulfiram in the blood stream presents a serious challenge for clinical application of disulfiram in cancer treatment and explains the lack of encouraging results from several clinical trials (http://www.clinicaltrials.gov/). The discrepancy between *in vitro* cytotoxicity and anticancer efficacy in patients indicates that the rapid degradation of disulfiram in the gastrointestinal system, hepatic first-pass effect and rapid metabolism in the blood stream may prevent a therapeutic amount of drug reaching the cancer, thus presenting a major limitation for clinical use of disulfiram in cancer treatment.

According to a first aspect of the invention, there is provided a disulfiram formulation, comprising diethyldithiocarbamic acid or diethyldithiocarbamate (DDC) together with a component that increases the *in vivo* half life of the disulfiram, diethyldithiocarbamic acid or diethyldithiocarbamate (DDC) for use in the treatment of cancer , wherein the component that increases the half life of the disulfiram, diethyldithiocarbamic acid or diethyldithiocarbamate (DDC) is a liposome or a nanoparticle and wherein the disulfiram formulation is formulated for simultaneous or sequential administration with a separate copper containing formulation .

Disulfiram has the chemical formula:

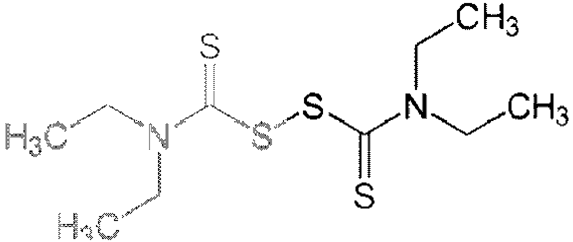

Preferably the activity of disulfiram, diethyldithiocarbamic acid or diethyldithiocarbamate (DDC) is maintained *in vivo* at at least 50% of its activity level prior to administration for at least about 2 hours post administration.

Preferably the *in vivo* half life of disulfiram, diethyldithiocarbamic acid or diethyldithiocarbamate (DDC) is increased to be at least 30 minutes, preferably at least one hour, more preferably at least 2 hours, more preferably at least 2.5 hours.

Preferably, the *in vivo* half-life refers to the time taken for half of an administered amount of disulfiram, diethyldithiocarbamic acid or diethyldithiocarbamate (DDC) to be degraded. Preferably degraded derivatives of disulfiram, or a derivative thereof, do not have any significant anti-cancer activity.

The *in vivo* half life may be measured by determining activity in plasma. This may be achieved by taking plasma samples from an individual over time.

The half life of the disulfiram, diethyldithiocarbamic acid or diethyldithiocarbamate (DDC) may be increased by encapsulating the disulfiram, diethyldithiocarbamic acid or diethyldithiocarbamate (DDC) .

Liposomes are artificial vesicles comprising an outer lipid bilayer, which in this case, would encapsulate the disulfiram, diethyldithiocarbamic acid or diethyldithiocarbamate (DDC)or a derivative thereof.

A liposome for use in the invention may have a diameter of from about 100nm to about 300nm, preferably about 175nm ± about 60nm.

Liposomes tend to be hydrophobic. In an alternative embodiment the component that increases the half life of the disulfiram may be a hydrophilic coating.

The liposomes may be characterized by a lipidic concentration of between 10 and 100mg/ml, and preferably around 50mg/ml.

The lipids in the liposome may be phospholipids, the phospholipids may be natural and/or synthetic in origin. Generally phospholipids are included that have a net neutral and/or a negative charge together with a sterol such as cholesterol. The phospholipids present may be phosphatidylcholine, which does not show any net charge and another phospholipid charged negatively, for example phosphatidylglycerol. The selection of lipid is made on the basis of the desired size of the final liposome, the drug to be encapsulated and the desired stability of the resulting liposome.

If cholesterol is included the proportion of cholesterol, with respect to the total quantity of lipids, may be between 0 and 50%, preferably between 35 and 50%. The cholesterol may increase the stability of liposomes by decreasing the permeability of the lipid bilayer to ions and small polar molecules, and/or by reducing the capacity of proteins to penetrate the bilayer and create disorder and thus instability.

Preferably in a formulation according to the invention comprising liposomes encapsulating at least disulfiram, diethyldithiocarbamic acid or diethyldithiocarbamate (DDC) the liposomes are stable and reproducible. Preferably the liposomes are stable in suspension. Preferably the liposomes are usable in cancer therapy. Preferably the liposomes are suitable for intravenous administration. Alternatively, the liposomes may be for oral administration.

Liposomes may be prepared by using the method of Bangham et al. in J.Mol. Biol. (1964) 8, 660-668, in which multilamenar liposomes (MLVs) are obtained of heterogeneous size. In this method the formative lipids are dissolved in a suitable organic solvent which later is eliminated by rotary evaporation under vacuum. The lipid film formed is subjected to hydration with a suitable aqueous medium containing the drug, by means of manual or mechanical agitation. The heterogeneous suspension of MLVs is subject to any of the known techniques of reduction and homogenization of sizes. For example, two preferred procedures are the sonication with titanium probes or extrusion through filters of polycarbonate of the solution of MLVs in order to obtain VET liposomes.

The component that increases the half life of the disulfiram, diethyldithiocarbamic acid or diethyldithiocarbamate (DDC) may alternatively be a nanoparticle (Malam et al (2009) Trends in Pharmacological Sciences Vol, 30 No. 11). Nanoparticles are particles ranging in size from 1 to 1000nm. The nanoparticle may be an albumin based nanoparticle. The nanoparticles may be solid lipid nanoparticles, also referred to as lipospheres, these are solid lipids at human physiological temperature (37°C) and have a diameter of 50 to 1000nm. They may be formed from a range of lipids, including mono, di and triglycerides, fatty acids, waxes and combinations thereof. Solid lipid nanoparticles form a strongly lipophilic matrix into which drugs can be loaded for subsequent release.

Alternatively, the nanoparticles may be polymer based nanoparticles. Usually such particles are formed from a biodegradable monomer based on simple organic molecules. The most widely used polymers are polylactide, poly(D, L-lactide-co-glycolide) and PEG. The liposomes may have surface modifications. This includes incorporation of proteins into the surface, binding of proteins and peptides to liposomes via amino acid groups, binding of proteins and peptides to liposomes via sulfhydryl groups, binding of carbohydrates and other small molecules to the liposomal surface, binding some antibodies or other cancer-targeting molecules to the liposomal surface.

In a still further alternative embodiment the nanoparticles may be gold nanoparticles comprising a core of gold atoms that can be functionalised by addition of a monolayer of moieties containing a thiol group.

The component that increases the half life of the disulfiram, diethyldithiocarbamic acid or diethyldithiocarbamate (DDC) may be a mesoporous silica nanoparticle (Coti et al (2009) Nanoscale Vol. 1, No.1, Pages 1-172).

The disulfiram, diethyldithiocarbamic acid or diethyldithiocarbamate (DDC) may be administered at a dose of about 250 - about 500 mg/day. Preferably, the disulfiram, diethyldithiocarbamic acid or diethyldithiocarbamate (DDC) is administered intravenously. 250 to about 500 mg/day of disulfiram is the tolerable dose used for alcoholism patients. Preferably, the patients are also administered about 2 mg of copper as copper gluconate. Preferably the copper gluconate is administered orally.

The disulfiram, diethyldithiocarbamic acid or diethyldithiocarbamate (DDC) may be administered at a dose of between about lmg/kilo and about 50mg/kilo, preferably at between about lmg/kilo and about 20mg/kilo, more preferably at about 10mg/kilo.

Preferably in a liposome according to the invention the disulfiram, diethyldithiocarbamic acid or diethyldithiocarbamate (DDC) has a longer *in vivo* half life than disulfiram, diethyldithiocarbamic acid or diethyldithiocarbamate (DDC) alone.

In a preferred embodiment the invention provides a liposome in which the only active ingredient encapsulated is disulfiram, diethyldithiocarbamic acid or diethyldithiocarbamate (DDC). Preferably the liposome does not encapsulate copper

Preferably a liposome encapsulating disulfiram, diethyldithiocarbamic acid or diethyldithiocarbamate (DDC)or a derivative thereof is stable at room temperature for at least one year.

The formulation of the invention may be used to kill and/or inhibit the replication of cancer cells and/or cancer stem cells.

The liposome encapsulation of disulfiram has been found to be surprisingly effective in killing or reducing the replication of cancer stem cells. Cancer stem cells are believed to be responsible for the failure of many cancer therapies that are ineffective against them.

The cancer can be any suitable cancer, for example, renal cancer, bladder cancer, ovarian cancer, breast cancer, endometrial cancer, pancreatic cancer, lymphoma, thyroid cancer, bone cancer, CNS cancer, leukaemia, liver cancer, prostate cancer, lung cancer, colon cancer, rectal cancer, brain cancer or melanoma.

The present application discloses a method of treating cancer comprising administering to a subject a therapeutically effective amount of a disulfiram formulation according to the invention.

### Uses of the formulation

The description discloses a method for treating or preventing cancer, the method comprising administering to a patient in need thereof a therapeutically effective amount of a formulation of the invention.

It will be appreciated that the term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The patient to be treated is preferably a mammal, in particular a human being, but it may also include animals, such as dogs, cats, horses, cows, sheep and pigs.

### Combination Therapies

Many diseases are treated using more than one medicament in the treatment, either concomitantly administered or sequentially administered.

It is therefore within the scope of the invention to use a formulation of the invention in a therapeutic method for the treatment of cancer as an adjunct to, or in conjunction with, other established therapies normally used in the treatment of the cancer. By analogy, it is also within the scope of the invention to use a formulation of the invention in combination with other therapeutically active compounds normally used in the treatment of cancer in the manufacture of a medicament for the treatment of cancer. Furthermore, by analogy, the invention also provides a formulation according to the invention in combination with other therapeutically active compounds normally used in the treatment of cancer, for use in the treatment of cancer.

In one embodiment a formulation of the invention may be for use in combination with surgery or radiotherapy, and/or with one or more of a Gliadel® Wafer (carmustine), 5-fluorouracil, doxorubicin, paclitaxel and gemcitabine. Preferably this combination is used in the treatment of one or more of colon cancer, breast cancer and glioblastoma (brain cancer). All the aforementioned examples show a very strong synergistic effect.

The combination treatment may be carried out in any way deemed necessary or convenient by the person skilled in the art and for the purpose of this specification, no limitations with regard to the order, amount, repetition or relative amount of the compounds to be used in combination is contemplated.

### Pharmaceutical Compositions

The medical treatment according to the invention is carried out with a pharmaceutical composition comprising disulfiram, diethyldithiocarbamic acid or diethyldithiocarbamate (DDC), or a pharmaceutically acceptable salt, solvate or hydrate thereof, as set out in the claims.

In one embodiment, there is provided a pharmaceutical composition as defined in the claims comprising disulfiram, diethyldithiocarbamic acid or diethyldithiocarbamate (DDC), or a pharmaceutically acceptable salt solvate or hydrate thereof for use in the treatment of cancer.

In the present context, the term "pharmaceutically acceptable salt" is intended to indicate salts which are not harmful to a patient. Such salts include pharmaceutically acceptable acid addition salts, pharmaceutically

In one embodiment a formulation of the invention may be for use in combination with surgery or radiotherapy, and/or with one or more of a Gliadel® Wafer (carmustine), 5-fluorouracil, doxorubicin, paclitaxel and gemcitabine. Preferably this combination is used in the treatment of one or more of colon cancer, breast cancer and glioblastoma (brain cancer). All the aforementioned examples show a very strong synergistic effect. The combination treatment may be carried out in any way deemed necessary or convenient by the person skilled in the art and for the purpose of this specification, no limitations with regard to the order, amount, repetition or relative amount of the compounds to be used in combination is contemplated.

### Pharmaceutical Compositions

In one embodiment, there is provided a pharmaceutical composition comprising disulfiram or a derivative thereof, or a pharmaceutically acceptable salt, solvate or hydrate thereof, together with a component that increases the *in vivo* half life of disulfiram or a derivative thereof, for use in the treatment of cancer.

In the present context, the term "pharmaceutically acceptable salt" is intended to indicate salts which are not harmful to a patient. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 1977, volume 66, issue 2. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995.

Suitable pharmaceutical carriers include inert solid diluents or fillers, sterile aqueous solutions and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. In addition, the compounds of the invention may form solvates with water or common organic solvents. Such solvates are also encompassed within the scope of the present invention.

The composition may further comprise a buffer system, preservative(s), tonicity agent(s), chelating agent(s), stabilizers and surfactants, which is well known to the skilled person. For convenience reference is made to Remington: The Science and Practice of Pharmacy, 20th edition, 2000. The composition may also further comprise one or more therapeutic agents active against the same disease state.

Methods to produce controlled release systems useful for compositions of the current invention include, but are not limited to, crystallization, condensation, co-crystallization, precipitation, co-precipitation, emulsification, dispersion, high pressure homogenisation, en-capsulation, spray drying, microencapsulating, coacervation, phase separation, solvent evaporation to produce microspheres, extrusion and supercritical fluid processes. General reference is made to Handbook of Pharmaceutical Controlled Release (Wise, D. L., ed. Marcel Dekker, New York, 2000) and Drug and the Pharmaceutical Sciences vol. 99: Protein Composition and Delivery (MacNally, E.J., ed. Marcel Dekker, New York, 2000).

Administration of pharmaceutical compositions according to the invention may be through several routes of administration, for example, oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route. It will be appreciated that the preferred route will depend on the general condition and age of the subject to be treated, the nature of the condition to be treated and the active ingredient chosen.

For topical use, sprays, creams, ointments, jellies, gels, inhalants, dermal patches, implants, solutions of suspensions, etc., containing the compounds of the present invention are contemplated. For the purpose of this application, topical applications shall include mouth washes and gargles. Compounds of the invention may be used in wafer technology, wafer technology, such as the biodegradable Gliadel polymer wafer, is very useful for brain cancer chemotherapy.

Pharmaceutical compositions for oral administration include solid dosage forms such as hard or soft capsules, tablets, troches, dragees, pills, lozenges, powders and granules and liquid dosage forms for oral administration include solutions, emulsions, aqueous or oily suspensions, syrups and elixirs, each containing a predetermined amount of the active ingredient, and which may include a suitable excipient.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations.

Tablets may contain the active ingredient in admixture with non-toxic pharmaceutically-acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example corn starch or alginic acid; binding agents, for example, starch, gelatin or acacia; and lubricating agents, for example magnesium stearate, stearic acid or talc.

The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356, 108; 4, 166,452; and 4,265,874 to form osmotic therapeutic tablets for controlled release.

Formulations for oral use may also be presented as hard gelatine capsules where the active ingredient is mixed with an inert solid diluent, for example, calcium carbonate, calcium phosphate or kaolin, or as soft gelatine capsules wherein the active ingredient is mixed with water or an oil medium, for example peanut oil, liquid paraffin, or olive oil.

Aqueous suspensions may contain the active compounds in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present.

The pharmaceutical compositions of the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, a preservative and flavouring and colouring agents. The pharmaceutical compositions may be in the form of a sterile injectable aqueous or oleaginous suspension. This suspension may be formulated according to known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as a solvent or suspending medium. For this purpose, any bland fixed oil may be employed using synthetic mono- or diglycerides. In addition, fatty acids such as oleic acid find use in the preparation of injectables.

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of the prolactin receptor antagonist in the form of a nasal or pulmonal spray. As a still further option, the pharmaceutical compositions containing the compound of the invention can also be adapted to transdermal administration, e.g. by needle-free injection or from a patch, optionally an iontophoretic patch, or transmucosal, e.g. buccal, administration.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use.

The term "aqueous composition" is defined as a composition comprising at least 50% w/w water. Likewise, the term "aqueous solution" is defined as a solution comprising at least 50% w/w water, and the term "aqueous suspension" is defined as a suspension comprising at least 50% w/w water. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art. Depot injectable formulations are also contemplated as being within the scope of the present invention.

When the use of the invention is combined with a second therapeutic agent active against the same disease state, they may conveniently be administered alone or in combination, in either single or multiple doses, sequentially or simultaneously, by the same route of administration, or by a different route.

Generally, depending on the intended mode of administration, the formulation will contain about 0.005% to 95%, preferably about 0.5% to 50%, by weight of a formulation of the invention. The percentage of active compound contained in the formulation is dependent on the specific nature of the formulation, as well as the activity of the compound and the needs of the subject. Percentages of active ingredient of 0.01% to 90% in solution are generally employable, whilst the amounts can be higher if the formulation is a solid which will be subsequently diluted. In some embodiments, the formulation will comprise from 1% to 50% of the active compound in solution.

### Effective Dosages

The use of a formulation according to the invention will generally be in an amount effective to achieve the intended result, for example in an amount effective to treat or prevent the particular disease being treated. The formulation may be administered therapeutically to achieve therapeutic benefit. By therapeutic benefit is meant eradication or amelioration of the underlying disorder being treated and/or eradication and/or amelioration of one or more of the systems associated with the underlying disorder. Therapeutic benefit also includes halting or slowing the progression of the disease, regardless of whether improvement is realised.

The exact dosage will depend upon the frequency and mode of administration, the sex, age, weight and general condition of the subject treated, the nature and severity of the condition treated and any concomitant diseases to be treated and other factors evident to those skilled in the art. Determination of the effective dosage is well within the capabilities of those skilled in the art.

When a formulation of the invention is used in combination with a second therapeutic agent active against the same disease state the dose of each compound may differ from that when the formulation is used alone. Appropriate doses will be readily appreciated by those skilled in the art.

In one preferred embodiment, the formulation will take the form of a unit dosage form. For example, the formulation may be provided in a vial or other container. The vial or other container may contain the formulation in the form of a liquid, a solid to be suspended, a dry powder, a lyophilisate, or any other suitable form.

It will be appreciated that optional features applicable to one aspect or embodiment of the invention can be used in any combination, and in any number. Moreover, they can also be used with any of the other aspects or embodiments of the invention in any combination and in any number. This includes, but is not limited to, the dependent claims from any claim being used as dependent claims for any other claim in the claims of this application.

The invention will be further described, by means of non-limiting example only, with reference to the following figures and experimental examples.

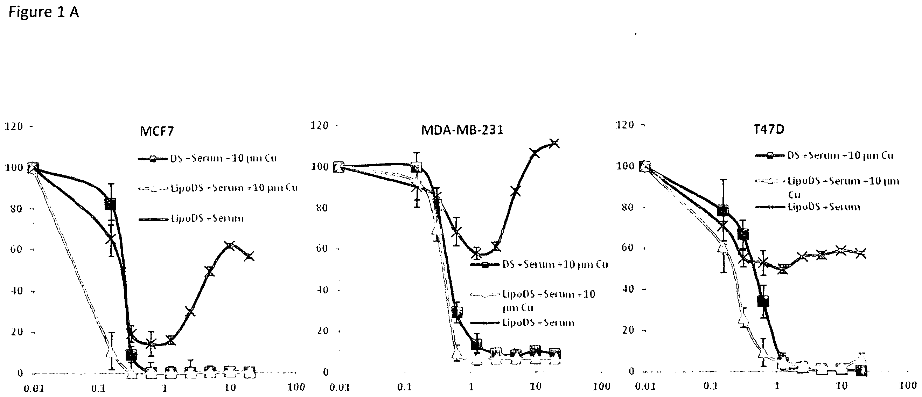

**Figure 1A to 1C** - demonstrate that liposomal disulfiram induces breast cancer cell apoptosis in a copper-dependent manner.
**Figure 1A** shows the results of an MTT cytotoxicity assay. The X axis is the drug concentration and the Y axis is the % survival. The breast cancer cell lines were exposed to different treatment for 72 hours and results show that the cytotoxicity of Lipo-DS (liposomal disulfiram)/Cu and DS/Cu was comparable. Without copper supplement, Lipo-DS failed to eliminate the cancer cells and the biphasic effect (second survival peak) was identified in two cell lines.

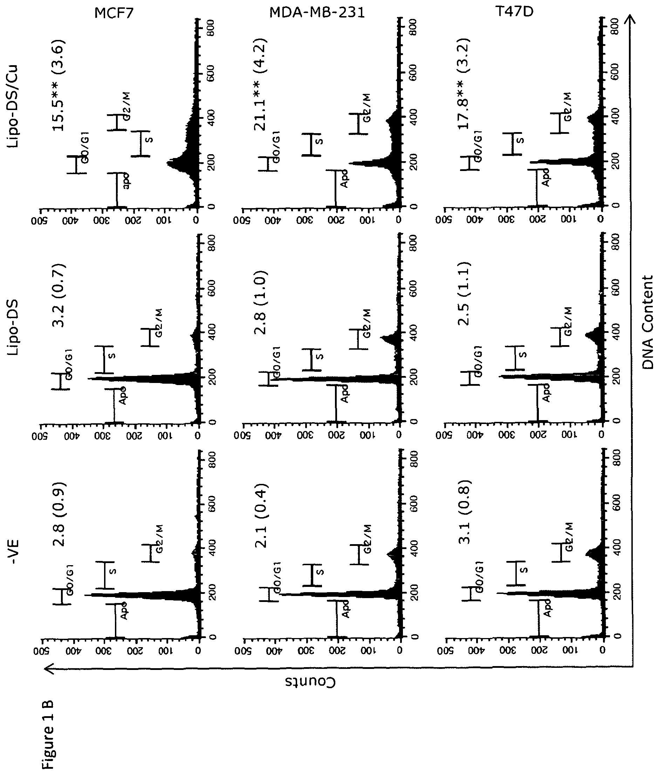

**Figure 1B** demonstrates the DNA content of cells following liposomal disulfiram treatment as analyzed by flow cytometry. Overnight cultured breast cancer cells were exposed to Lipo-DS (0.5 *µ*M) or Lipo-DS (0.5 *µ*M) plus Cu (10 *µ*M) for 72 hours. The DNA contents in the treated cells (10,000 events) were determined. The sub-G1 population represents the apoptotic cells (** p < 0.01, n = 3).

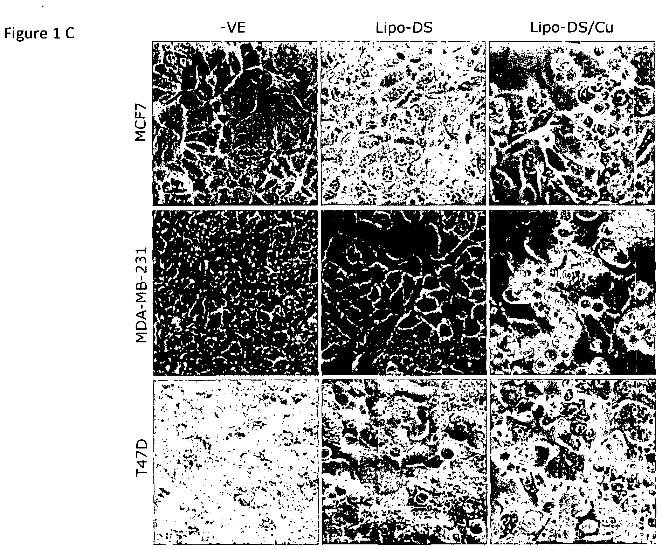

**Figure 1C** illustrates the morphology (× 100 magnification) of breast cancer cell lines after 72 hours drug exposure.

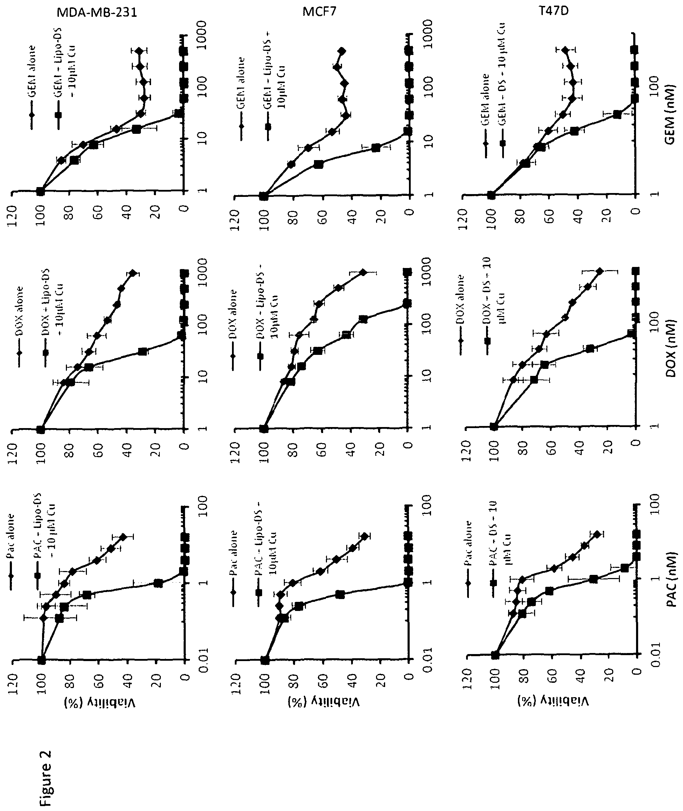

**Figure 2** - illustrates the results of MTT analysis which demonstrate the enhancing effect of Lipo-DS on paclitaxel (PAC), gemcitabine (GEM) and doxorubicin (DOX). DOX:Lipo-DS = 1:5, GEM:Lipo-DS = 1:10, PAC:Lipo-DS = 1:62.5. The cells were exposed to conventional anticancer drugs or anticancer drugs in combination with Lipo-DS/Cu10*µ*M for 72 hours before being subjected to MTT assay.

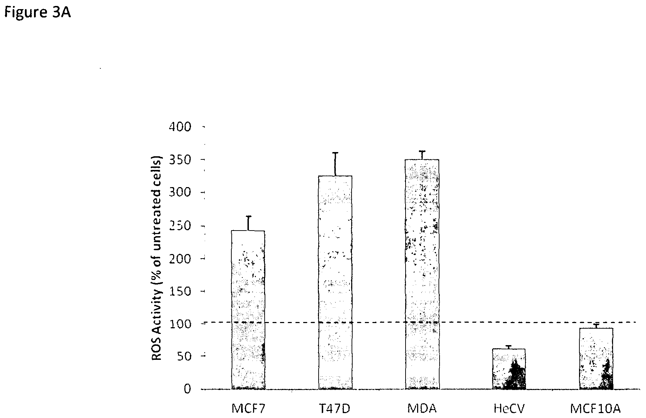

**Figure 3A** - demonstrates that reactive oxygen species (ROS) are responsible for the cytotoxicity and selectivity of Lipo-DS/Cu in breast cancer cell lines. The results demonstrate Lipo-DS/Cu induced ROS generation in breast cancer but not normal cells. The breast cancer and normal cell lines were loaded with ROS detection solution and exposed to Lipo-DS (1*µ*M) and Cu (10*µ*M) for 1 hour. The ROS positive population was detected by flow cytometry. The figure demonstrates the mean and SD of the relative ROS activity [(Treated/untreated) × 100%] from three experiments.

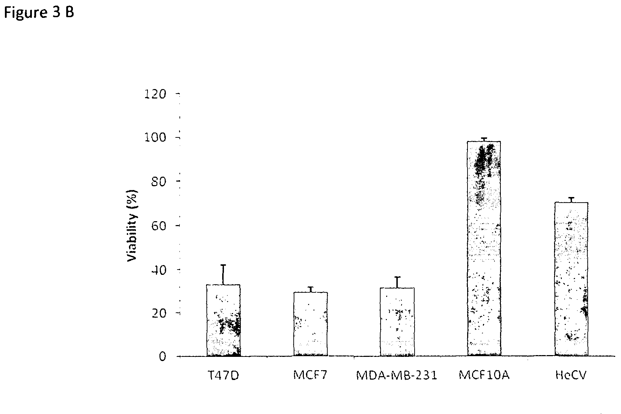

**Figure 3B** **-** demonstrates the selective targeting effect of Lipo-DS/Cu on breast cancer cell lines. The breast cancer (T47D, MCF7 and MDA-MB-231) and normal (MCF10A and HeCV) cell lines were exposed to Lipo-DS/Cu. After 72 hours, the viability of the treated cells was determined by MTT assay.

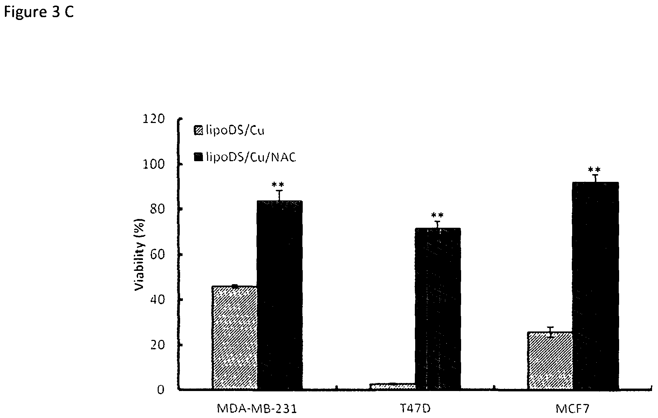

**Figure 3C** - demonstrates that the ROS inhibitor (NAC) reverses Lipo-DS/Cu induced cytotoxicity in breast cancer cell lines. The breast cancer cell lines were exposed to Lipo-DS/Cu with or without NAC (10mM) for 72 hours. The viability was determined by MTT assay.

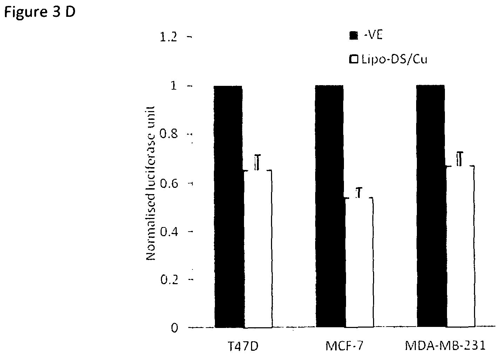

**Figure 3D** - demonstrates that Lipo-DS/Cu inhibited NF B DNA binding activity detected by EMSA assay. The breast cancer cell lines were treated with Lipo-DS1*µ*M/Cu10*µ*M for 24 hours.

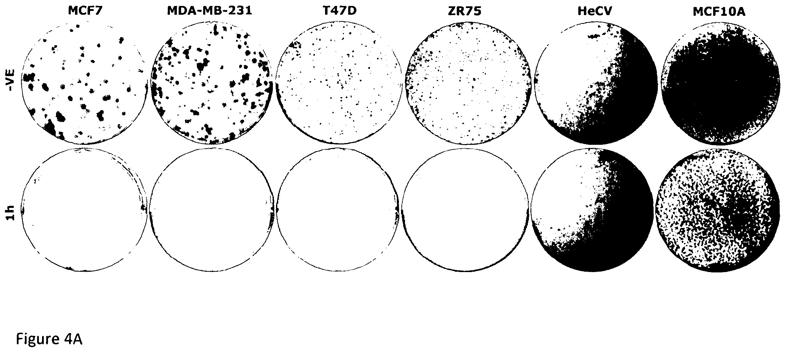

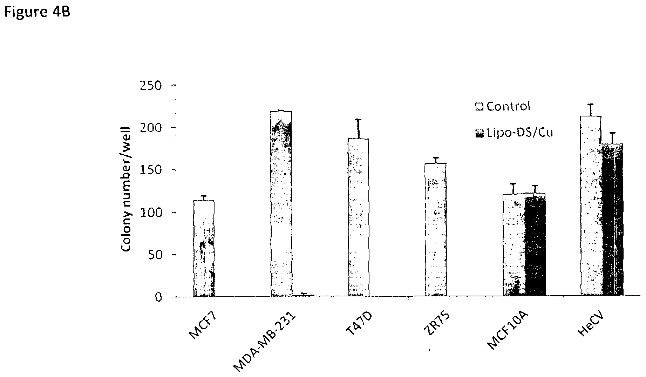

Figure 4A and 4B - illustrate the effect of DS/Cu on the clonogenicity of breast cancer cell lines (MCF7, MDA-MB-231, T47D and ZR75) and normal cell lines (MCF10A and HeCV). The cells were exposed to Lipo-DS (1*µ*M)/Cu (10*µ*M) for one hour. The drug treated cancer (2.5 × 10³) and normal (5 × 10³) cells were resuspended in drug free medium and plated onto 6-well plates. After 10 days culture, the colony (> 50 cells) number was counted. **Figure 4A** shows typical clonogenic assay images, **Figure 4B** shows the mean and SD of the colony number from three experiments.

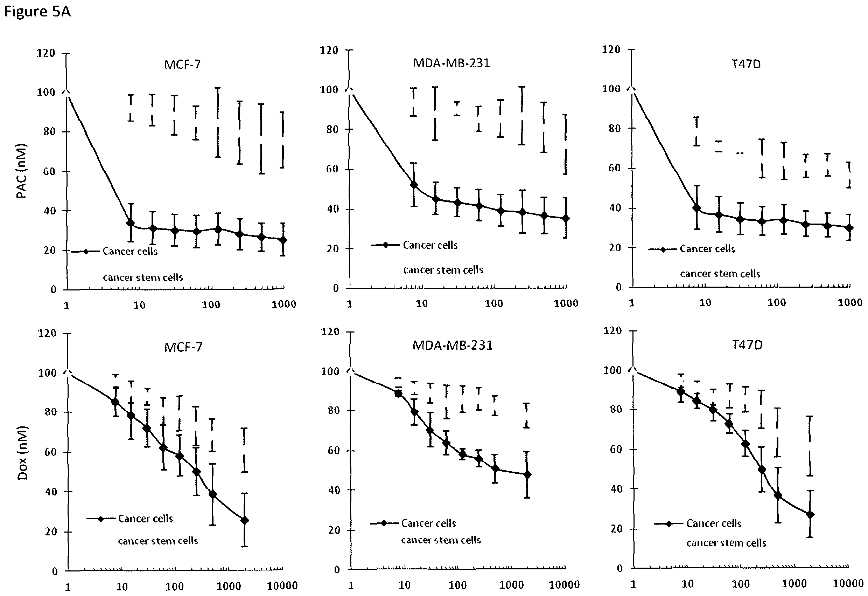

**Figures 5A to 5E** demonstrate the effect of Lipo-DS/Cu on cancer stem cells.

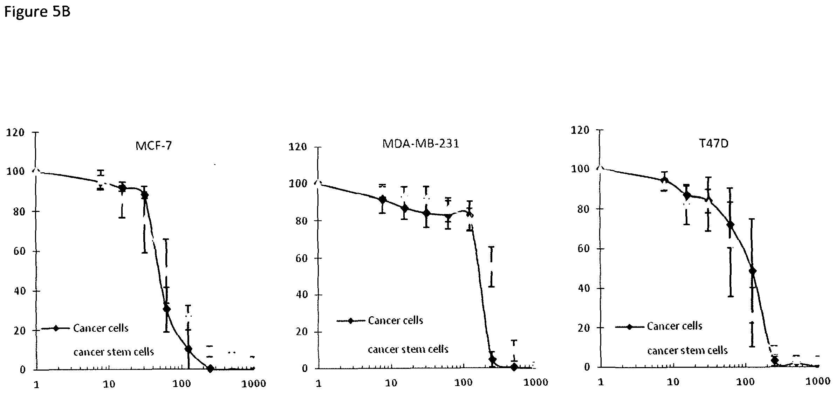

**Figures 5A** **and** **5B** illustrate the cytotoxicity of conventional anticancer drugs and Lipo-DS/Cu in cancer stem cells (CSCs) and normal cancer cells. CSCs were highly resistant to conventional anticancer drugs but not Lipo-DS/Cu. The CSCs were enriched by culturing in ultralow attachment plates containing stem cell medium. The CSCs and normal cancer cells were exposed to Lipo-DS/Cu for 72 hours and the viability was determined by MTT assay.

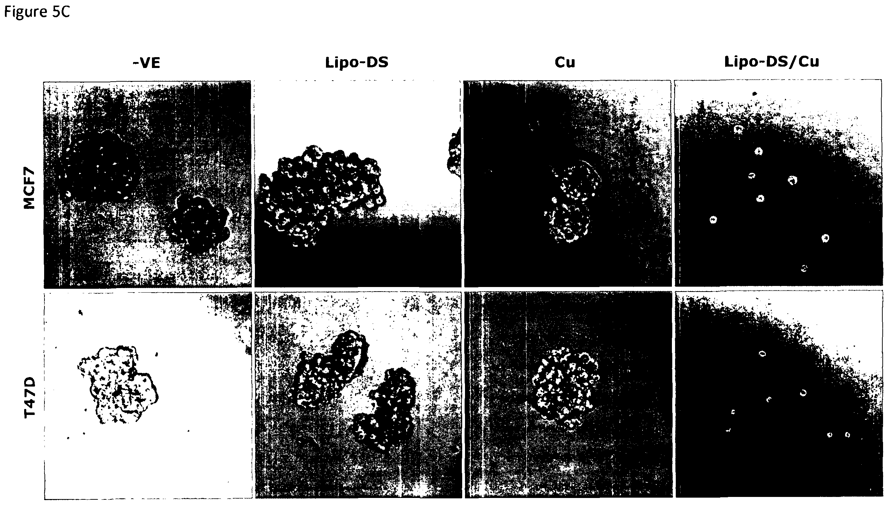

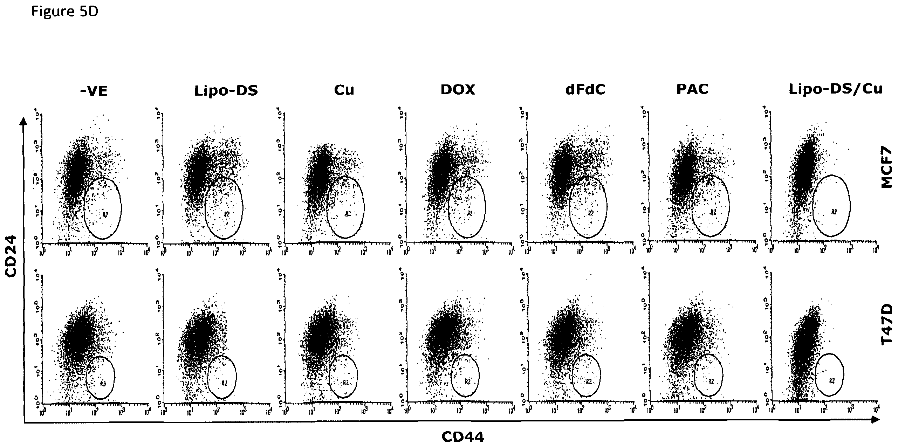

**Figures 5C** **and** **5D** illustrate that the CSCs (ALDH1 and CD24Low/CD44High) population was abolished by Lipo-DS/Cu. The CSCs were exposed to Lipo-DS (1*µ*M)/Cu (10*µ*M) for one hour and recovered in drug free medium for 16 hours. The ALDH1 and CD24Low/CD44High CSC population was determined by flow cytometry.

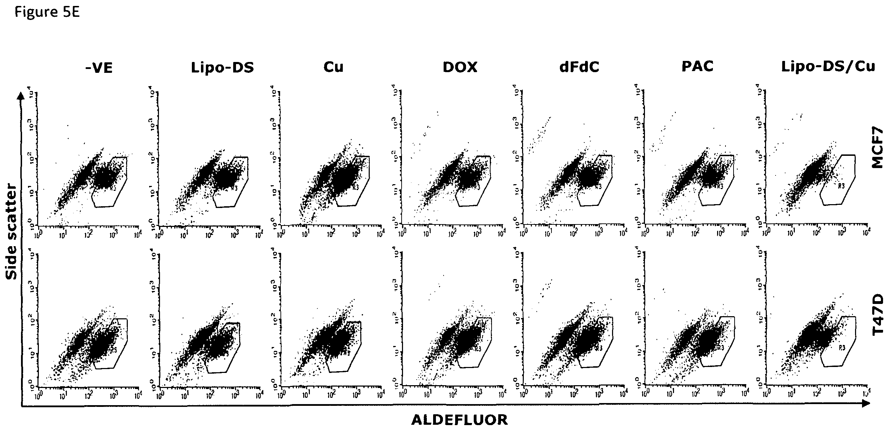

**Figure 5E** **-** Demonstrates that Lipo-DS/Cu inhibits mammosphere formation. Breast cancer cells were treated with Lipo-DS1*µ*M, Cu10*µ*M or Lipo-DS/Cu for 1 hour then sub-cultured in drug-free stem cell medium in ultra-low attachment 96-well plates (30 cells/well) for 10 days. The mammosphere (> 50 cells/sphere) number was counted from 30 wells and the image was photographed at × 40 magnification.

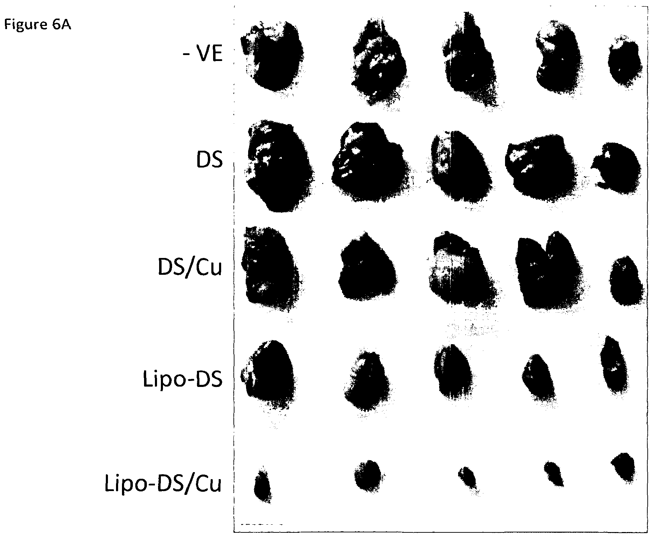

**Figures 6A to 6F** demonstrate that Lipo-DS/Cu inhibits tumour growth in mice.

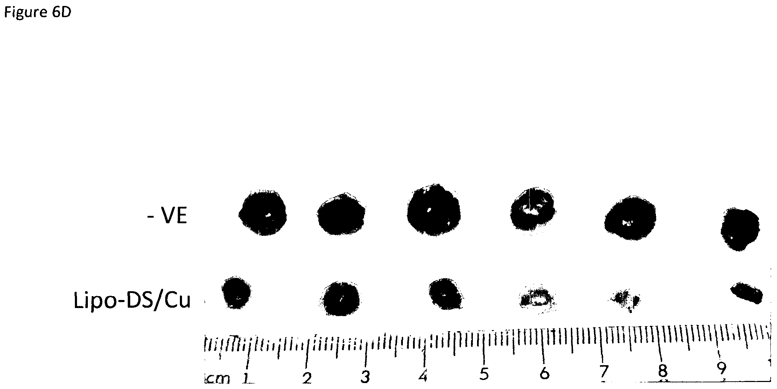

An S180 mouse sarcoma (**Figures 6A**-**6C**) and MDA-MB-231 breast cancer cells (1 × 10⁶) (**Figure 6D-6F**) were subcutaneously injected into the rare frank of BALB/c and CD1 Nu/Nu mice respectively (S180: n=5; MDA-MB-231: n=6). When the xenograft reached 2 mm in diameter, the tumour bearing mice were subjected to the following treatment.
1) Control: no treatment (-ve)
2) Lipo-DS: 30mg/kg i.v. (lipo-DS)
3) Lipo-DS i.v. + CuCl₂ oral: CuCl₂ 3mg/kg p.o. first and then Lipo-DS 30mg/kg i.v. (lipo-DS/Cu)
4) DS free drug (30mg/kg) oral (S180 experiment only). (DS)
5) DS free drug (30mg/kg) oral + CuCl₂ (3mg/kg) oral (S180 experiment only). (DS/CU)

The drugs were administrated on Monday, Wednesday and Friday for two successive weeks and the animals were observed for another one and two weeks for breast cancer and S180 sarcoma respectively.

### Example 1

### Preparation of the liposomes encapsulating disulfiram

Liposomes encapsulating disulfiram are prepared by preparing a chloroform solution containing appropriate lipids, for example those listed in table 1 below, in a round-bottomed flask. Afterward the organic solvent is eliminated by means of roto-evaporation, additionally traces of solvent are eliminated by passage of N₂ or by lyophilisation. The lipidic film thus obtained is then hydrated with 3 ml of NaCl solution at 0.9% containing disulfiram, by agitation in a vibromixer at intervals of 30 sec, followed by resting in a bath maintained at 60°C for the same amount of time and an effective agitation up to 10 min. This will form a liposomal dispersion containing preferably multilamelar vesicles (MLVs). The liposomal solution is then sonicated by ultrasonic irradiation using a titanium probe until a change in the turbidity in observed. The protocol of sonication is carried out in relays of 2 min of sonication and one minute resting under atmosphere of N₂ in an ice water bath. In order to eliminate the particles of titanium produced during the sonication, the resulting solution is centrifuged for 20 min. at 3000 rpm. The liposomal suspension thus obtained preferably then contains small unilamelar liposomes (SUVs).

Alternatively, a suspension of multilamelar liposomes obtained using the above method are extruded through polycarbonate filters with 0.4 and 0.2µm diameter pores to obtain liposomes designated VET.

The percentage of encapsulation of disulfiram is determined by chromatographic separation of a portion of the liposomal suspension using Sephadex G-50M and NaCl 0.9% as eluent.

**Table I**

| TYPE | COMPOSITION | RATIO (mg) |
|---|---|---|
| MLV | PC:PG:CHOL:DSF | 50:50:50:15 |
| SUV | PL:PG:CHOL:DSF | 50:50:50:15 |
| SUV | PC:PG:CHOL:DSF | 50:50:50:15 |
| SUV | DPPC:DPPG:CHOL:DSF | 50:50:50:15 |
| VET | PL:PG:CHOL:DSF | 50:50:50:15 |
| VET | PC:PG:CHOL:DSF | 50:50:50:15 |
| VET | DPPC:DPPG:CHOL:DSF | 50:50:50:15 |

In Table I the following acronyms are used: MLV-multilamelar vesicles; SUV-small unilamelar liposome; VET-extruded multilamelar liposomes; PC-phosphatidylcholine; PG-phosphatidylglycerol; CHOL-cholesterol; DSF - disulfiram.

In another embodiment disulfiram lipid micropsheres (liposomes) wers prepared using the following components:

| | |
|---|---|
| Disulfiram (DS) | 0.3g |
| Soybean lecithin | 1.2g |
| Medium chain triglyceride (MCT) | 10g |
| F-68 | 0.2g |
| Sodium oleate | 0.025g |
| Glycerine | 2.5g |
| Water for injection to make | 100ml |

Soybean lecithin was dispersed in MCT at 70°C and then mixed with DS until dissolved in an oil phase. The aqueous phase consisting of F68, sodium oleate and glycerine were heated in a water bath at 65°C until uniformly dispersed. Afterwards, the pH value of the aqueous phase was adjusted to 6.0 with 0.1mol/L NaOH and H₃PO₄. The coarse emulsion was prepared by adding the aqueous water to the oil phase rapidly with high shear mixing at 8000rpm. The high shear mixing process was carried out for 2 minutes and repeated twice. Then the volume of coarse emulsion was adjusted to 100ml with water for injection. The final emulsion was obtained by high-pressure homogenisation at 700bar for eight cycles. The temperature of the entire homogenisation process was controlled below 40°C in an ice bath. Finally the emulsion was transferred to vials followed by adding nitrogen gas and autoclaved at 121°C for 10 mins.

Liposomes containing disulfiram made by the above method were shown to be stable of plasma for at least 25 hours, with about 75% remaining after 25 hours, whereas free disulfiram was rapidly degraded in plasma, with only about 15% remaining after 25 hours.

Similarly, in whole blood liposomes containing disulfiram made by the above method were shown to be stable for at least 5 hours, with about 50% remaining after 5 hours, whereas free disulfiram was rapidly degraded in whole blood, with only about 10% remaining after 5 hours.

The effect of the oil used to produce the liposomes was considered, in particular LCTs and MCTs alone or in combination were considered. The stability of disulfiram in liposomes made with MCT alone, LCT alone and a mixture of MCT and LCT (1:1) was assessed. The oil containing disulfiram were placed in an oven at 60°C and the changes in content were measured. The results are shown in Table 1A.

**Table 1A - Drug remaining of disulfiram (%) in different oil phases at 60°C during 10 days**

| Oil | 5th day at 60°C | 10th day at 60°C |
|---|---|---|
| MCT | 97.37 | 85.05 |
| MCT:LCT = 1:1 | 83.75 | 70.66 |
| LCT | 67.47 | 51.94 |

The results show that the content of disulfiram in MCT reduced rather less than that in LCT and the mixture of two kinds of oil. MCTs have been reported to be a better solubilizer and exhibit greater oxidative stability compared to LCTs. In addition, MCTs have been investigated to be better solubilizers of disulfiram than LCTs.

### Example 2

### Liposomal disulfiram (Lipo-DS) induces breast cancer (BC) cell apoptosis in a Cu-dependent manner.

The cytotoxicity of DS and Lipo-DS to breast cancer cell lines was compared. In CuCl₂ (10 *µ*M) containing medium, both Lipo-DS and DS showed comparable cytotoxicity to BC cell lines (Figure 1A). Without copper supplement, Lipo-DS could not completely eradicate the cancer cells and the cytotoxicity assay demonstrated a biphasic effect in two breast cancer cell lines. The flow cytometric DNA content analysis demonstrated significant increase of apoptosis (sub-G1 population) after 72 hours Lipo-DS/Cu exposure (Figure 1B). The drug-induced morphological changes are showed in Figure 1C.

### Example 3

### Lipo-DS/Cu synergistically enhances the cytotoxicity of PAC, Dox and dFdC in BC cell lines.

The enhancing effect of Lipo-DS on three first line anti-breast cancer drugs was examined. In combination with Lipo-DS/Cu10*µ*M, the cytotoxicity of PAC, Dox and dFdC in three breast cancer cell lines was significantly enhanced (Figure 2 and Table 2).

**Table 2. Cytotoxic effect of single anticancer drug and in combination with Lipo-DS/Cu on MCF-7, MDA-MB-231 and T47D cell lines**

| Treatments | Ratio* | MCF7 | MDA-MB-231 | T47D |
|---|---|---|---|---|
| Dox alone | | 440.5 (16.2) | 178.5 (11.9) | 160.0 (5.5) |
| Dox + Lipo-DS/Cu¶ | 1:5 | 40.3 (9.3)** | 22.0 (2.0)** | 21.5 (3.1)** |
| GEM alone | | 22.1 (3.4) | 12.3 (2.2) | 35.0 (4.3) |
| GEM + Lipo-DS/Cu | 1:10 | 4.9 (0.5)** | 11.3 (1.2) | 12.4 (0.7)** |
| Pac alone | | 4.3 (1.4) | 9.3 (0.7) | 2.6 (0.3) |
| Pac + Lipo-DS/Cu | 1:62.5 | 0.4 (0.1)** | 0.6 (0.02)** | 0.7 (0.1)** |

| | | | | |
|---|---|---|---|---|
| *The ratio of anticancer drug: Lipo-DS. ¶Copper concentration was fixed at 10µM. The figures in the table represent the mean of the IC50 values calculated from 3 replicates with SD in parentheses. ** p < 0.01 | | | | |

The combination effect between Lipo-DS/Cu10*µ*M and these three conventional anticancer drugs was examined using CI-isobologram analysis which demonstrated high synergism between Lipo-DS/Cu10*µ*M and the conventional anticancer drugs over a wide range of concentrations (IC50 - IC90, Table 3).

**Table 3. Combination index (CI) § values of the interaction between anticancer drug and Lipo-DS/Cu in human breast cancer cell lines**

| Cell lines/Treatments | Ratio* | Combination index values | | |
|---|---|---|---|---|
| | | IC50 | IC75 | IC90 |
| MCF-7 | | | | |
| Dox + Lipo-DS/Cu | 1:5 | 0.513 | 0.499 | 0.502 |
| dFdC + Lipo-DS/Cu | 1:10 | 0.484 | 0.479 | 0.513 |
| Pac + Lipo-DS/Cu | 1:62.5 | 0.183 | 0.213 | 0.265 |

| MDA-MB-231 | | | | |
|---|---|---|---|---|
| Dox + Lipo-DS/Cu | 1:5 | 0.923 | 0.797 | 0.759 |
| dFdC + Lipo-DS/Cu | 1:10 | 0.554 | 0.601 | 0.832 |
| Pac + Lipo-DS/Cu | 1:62.5 | 0.437 | 0.436 | 0.457 |

| T47D | | | | |
|---|---|---|---|---|
| Dox + Lipo-DS/Cu | 1:5 | 0.655 | 0.54 | 0.521 |
| dFdC + Lipo-DS/Cu | 1:10 | 0.881 | 0.551 | 0.45 |
| Pac + Lipo-DS/Cu | 1:62.5 | 0.742 | 0.661 | 0.633 |

| | | | | |
|---|---|---|---|---|
| *Constant ratios of anticancer drug:Lipo-DS in nM. §Combination index (CI) values shown were from representative experiments repeated three times with similar results and were calculated on the multiple effect equation derived by Chou and Talalay (1984). CI < 1 indicates synergistic effect between two drugs. | | | | |

### Example 4

### Lipo-DS/Cu specifically induces ROS generation in breast cancer cell lines and the cytotoxicity of Lipo-DS/Cu is ROS-related

The ROS activity in breast cancer cell lines was markedly induced by exposure to Lipo-DS (1 *µ*M)/Cu (10 *µ*M) for one hour. In contrast, the ROS activity in normal cell lines (MCF10A and HeCV) was not affected or even reduced after drug exposure (Figure 3A). In line with the ROS activity results, 1 hour Lipo-DS (1 *µ*M)/Cu (10 *µ*M) exposure induced strong cytotoxicity in breast cancer cell lines but not normal cell lines (Figure 3B). To determine the relationship between ROS and Lipo-DS/Cu induced cytotoxicity, NAC, a ROS inhibitor, was used to block Lipo-DS/Cu induced ROS activity. Figure 3C shows that the Lipo-DS/Cu induced cytotoxicity in breast cancer cell lines was significantly reversed by addition of NAC in the culture.

### Example 5

### Lipo-DS/Cu inhibits NFKB activity in breast cancer cell lines

NFKB is a ROS-induced transcription factor with strong anti-apoptotic activity which in turn dampens the pro-apoptotic effect of ROS (Nakano et al 2006 Cell Death and Differentiation 13(5) 730-7). Blockage of NFKB activation enhances ROS-induced cytotoxicity. Figure 3D showed that the NFKB transcriptional activity in breast cancer cell lines was significantly inhibited by Lipo-DS/Cu.

### Example 6

### Lipo-DS/Cu inhibits the clonogenicity in breast cancer cell lines

Clonogenic assays (Franken et al., 2006 Nat Protoc, 1, 2315-9) were performed to examine the ability of Lipo-DS/Cu to induce 'reproductive death' in breast cancer cells. Figure 4A and B demonstrate that after only one hour exposure of breast cancer cell lines to Lipo-DS (1 *µ*M)/Cu (10 *µ*M), the clonogenicity in these cancer cell lines was completely abolished. In contrast, the drug exposure did not affect the clonogenicity in two normal cell lines.

### Example 7

### Lipo-DS/Cu targets breast cancer stem cells

Cancer stem cells (CSCs) are resistant to chemotherapy and responsible for chemotherapy failure and cancer recurrence. Figure 5A demonstrates that the CSCs derived from three breast cancer cell lines were highly resistant to conventional anticancer drugs. In contrast, Lipo-DS/Cu showed comparable cytotoxicity to both CSCs and their parental counterparts (Fig. 5B). This result indicates that Lipo-DS/Cu can effectively eradicate CSCs although CSCs are commonly resistant to a wide range of conventional anticancer drugs. Breast cancer cells can form mammospheres when cultured in stem cell medium. Mammospheres contain high percentage of breast cancer stem cells which can be detected by stem cell markers (ALDH positive and CD24Low/CD44High). Figure 5C and Table 4 demonstrate that the mammosphere forming ability in MCF7 and T47D cell lines was completely blocked after 1 hour exposure to Lipo-DS (1 *µ*M)/Cu (10 *µ*M). Lipo-DS and Cu single treatment had no effect on the mammosphere forming ability in these cell lines. To determine the targeting effect of DS/Cu on CSCs, the breast cancer stem cell markers in drug-treated mammosphere cells were also analyzed. Figure 5D and 5E demonstrate that the ALDH positive and CD24Low/CD44High CSC population in mammospheres was abolished after treated with Lipo-DS/Cu but not DS, Cu alone or conventional anticancer drugs.

**Table 4. The effect of Lipo-DS on mammosphere forming ability in breast cancer cell lines**

| | Control | Lipo-DS | Cu | Lipo-DS/Cu |
|---|---|---|---|---|
| MCF7 | 35.5 (0.7) | 41.5 (0.7) | 37.0 (1.4) | 0 |
| T47D | 58 (2.8) | 61 (2.8) | 52.5 (6.4) | 0 |

Lipo-DS 1*µ*M, Cu: CuC12 1*µ*M. The cells were exposed to different treatment for 1 hour and then cultured in drug free medium for another 10 days. The figure in the table represents mean from 3 experiments with SD in parentheses.

### Example 8

### Anti-tumour effect of Lipo-DS in tumour burdened mice

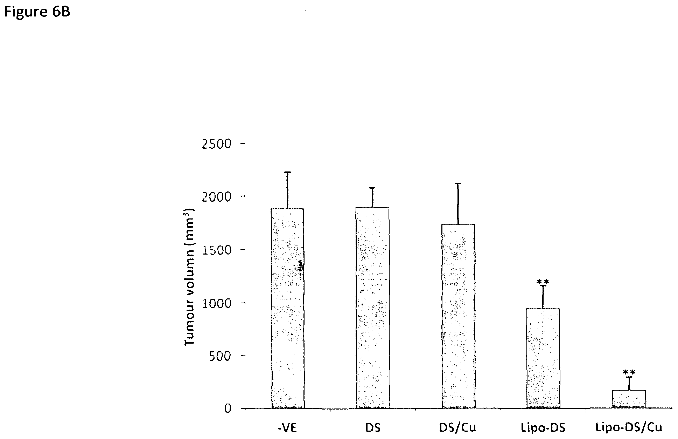

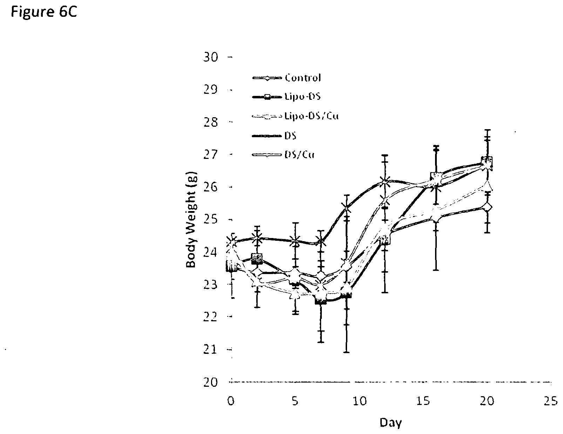

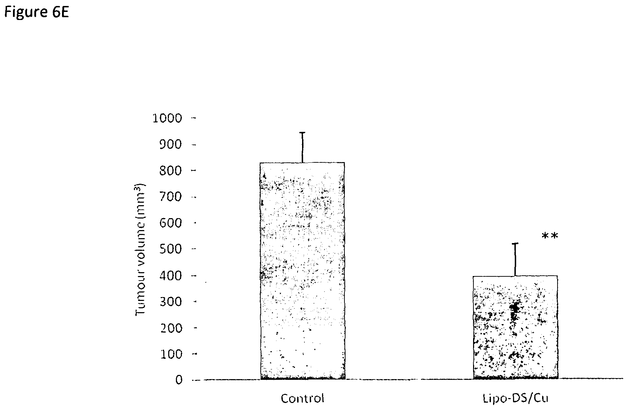

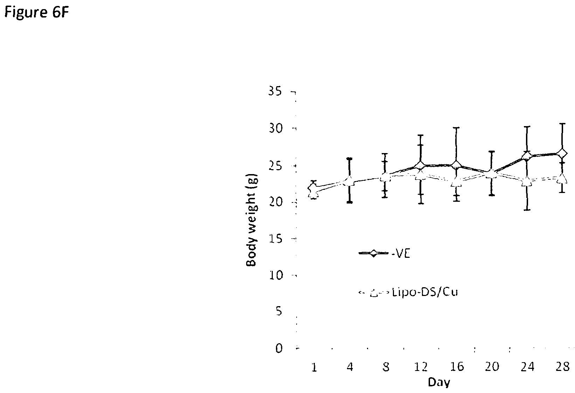

The anticancer effect of Lipo-DS was examined in S180 sarcoma bearing mice. Figures 6A and B show that administration of Lipo-DS and copper in combination significantly inhibited S180 sarcoma growth in mice. The inhibiting rate was 83%. Lipo-DS alone also significantly inhibited tumour growth although the inhibiting rate (52%) was lower than that in Lipo-DS/Cu treated group. In contrast the in vivo anticancer effect was not detected in the mice treated with DS free drug alone or in combination with copper. Furthermore we examined the anticancer effect of Lipo-DS/Cu on human breast cancer xenograft. Figure 6D and E show that in comparison with the control group, a 54% inhibition of tumour growth was observed in the Lipo-DS/Cu treated group. The systemic toxicity of Lipo-DS was determined by the effect on mouse body weight. As shown in Figure 6C and F there was no significant body weight difference between different treatment groups. These results indicate that Lipo-DS did not cause systemic toxicity to mice.

### Example 9

Toxic effect of Lipo-DS with and without copper on mice Table 5 demonstrates the acute toxic effect of liposomal disulfiram + copper glutanate on mice, Table 5 demonstrate the effect without copper.

**Table 5**

| Group | Dose (mg/kg) | Number of mice | Number of dead mice | Death rate (%) |
|---|---|---|---|---|
| Control | | | | |
| | 0 | 5 | 5 | 0 |

| Treatment | | | | |
|---|---|---|---|---|
| | 200 | 20 | 15 | 75 |
| | 170 | 20 | 9 | 45 |
| | 140 | 20 | 3 | 15 |
| | 120 | 20 | 2 | 10 |
| | 100 | 20 | 1 | 5 |

The copper gluconate (1mg/kg/day) was orally administered for 3 days in a row. After last dose of copper gluconate administration, different doses of liposomal disulfiram in 0.2 ml were administered via tail vein injection. The LD50 = 173.15 mg/kg.

**Table 6 Acute toxic effect of liposomal disulfiram without copper glutanate on mice**

| Group | Dose (mg/kg) | Number of mice | Number of dead mice | Death rate (%) |
|---|---|---|---|---|
| Control | | | | |
| | 0 | 5 | 5 | 0 |

| Treatment | | | | |
|---|---|---|---|---|
| | 200 | 20 | 15 | 75 |
| | 170 | 20 | 13 | 65 |
| | 140 | 20 | 2 | 10 |
| | 120 | 20 | 2 | 10 |
| | 100 | 20 | 0 | 0 |

Different doses of liposomal disulfiram in 0.2 ml were administered via tail vein injection without copper supplement. The LD50 = 168.77 mg/kg.

### Materials and Methods

**Cell lines and reagents.** The human breast cancer cell lines MCF7, MDA-MB-231, T47D, ZR75 and mouse S180 sarcoma cell line were purchased from ATCC. DS, copper (II) chloride (CuCl₂), N-acetylcysteine (NAC), paclitaxel (PAC), doxorubicin (Dox), gemcitabine (dFdC) were purchased from Sigma. The Lipo-DS injection was provided by Prof Xing Tang at Shenyang Pharmaceutical University, China.

**Cell Culture and Cytotoxicity Analysis.** All cell lines were cultured in DMEM (Lonza, Wokingham, UK) supplemented with 10% FCS, 50 units/ml penicillin, 50 µg/ml streptomycin. For in vitro cytotoxicity assay, cells [5,000/well (10,000/well for MDA-MB-231PAC10)] were cultured overnight in 96-well flat-bottomed microtiter plates then exposed to drugs for 72 h and subjected to a standard MTT assay (Plumb JA, et al Cancer Res 1989;49(16):4435-40).

**Analysis of the combinational effect of conventional anticancer drugs and DS/Cu by CI-isobologram.** Overnight cultured cells were exposed to various concentrations of conventional anticancer drugs (PAC, Dox, dFdC), Lipo-DS/Cu10*µ*M or in combination of conventional anticancer drug and Lipo-DS/Cu10*µ*M at a constant ratio of PAC:Lipo-DS = 1:62.5, dFdC:Lipo-DS = 1:10, Dox:Lipo-DS = 1:5 for 72 h. The cells were then subjected to MTT analysis as described above. The combinational cytotoxicity of PAC, dFdC, Dox and Lipo-DS/Cu10*µ*M was determined using CI-isobologram analyzed by CalcuSyn software (Biosoft, Cambridge, UK). The combination index (CI) was determined by mutually exclusive equations.

**Flow Cytometric Analysis of DNA Content.** Cells (1 × 10⁶) were exposed to drugs and harvested by trypsinisation. The cells were fixed in 70% ethanol and then incubated with RNase A (100 µg/ml) and propidium iodide (50 µg/ml) for 30 min. The data from 10,000 cells of each sample were collected by FACS Scan (Becton Dickinson, NJ) and the DNA content analyzed using CellQuest software (BD Biosciences, Oxford, UK).

**ROS activity detection.** The intracellular ROS levels were determined using 2',7'-dichlorodihydrofluorescein diacetate (H₂DCFDA) probe (Invitrogen, Paisley, UK). Cancer cells (1 × 10⁶) were cultured in 24-well plates with 1 ml of serum- and phenol red-free DMEM medium (Sigma) containing 20 *µ*M of H₂DCFDA. Fluorescence was measured at excitation 490 nm and emission 520 nm using a Fluoroskan Ascent fluorometer (Thermo Scientific, Northumberland, UK).

**Luciferase reporter gene assay.** All the transfections were performed using Lipofectamine 2000 (Invitrogen) transfection reagent following the manufacturer's instructions. The cells were co-transfected with luciferase reporter vectors [pNFκB-Tal-Luc (BD Biosciences) and pGL3-Basic (Promega, Southampton, UK)] and an internal control, pSV40-Renilla (Promega). The luciferase activities were determined using Dual Luciferase Assay reagents (Promega) following the manufacturer's instructions. The luciferase activity in each well was normalized to pSV40-Renilla using the formula of Ln = L/R (Ln: normalized luciferase activity; L: luciferase activity reading and R: Renilla activity reading). The Ln was further standardized by the transcriptional activity of the pGL3-Basic using the formula of RLU = LnNFκB/LnBasic (RLU: relative luciferase unit).

**Clonogenic Assay.** Cells (5 × 10⁴/well in 6-well plates) were exposed to Lipo-DS/Cu10*µ*M for 1 h. The cells were collected and further cultured for 10 days in 6-well plates containing drug-free medium at a cell density of 2.5 × 103/well. Clonogenic cells were determined as those able to form a colony consisting of at least 50 cells.

**Detection of ALDH positive population.** The ALDH positive population in drug-treated BC cell lines was detected by ALDEFLUOR kit (StemCell Tech., Durham, NC, USA) following the supplier's instruction. The cells (2.5 × 10⁵) were analyzed after stained in ALDH substrate containing assay buffer for 30 min at 37°C. The negative control was treated with diethylaminobenzaldehyde (DEAB), a specific ALDH inhibitor.

***In vitro* mammosphere culture.** The BC cells were cultured in Ultra-low adherence 6-well plates (Corning, MA, USA) containing 2 ml of stem cell culture medium [SCM, serum-free DMEM-F12 supplemented with B27 (Invitrogen, Paisley, UK), 20 ng/ml epidermal growth factor (Sigma), 10 ng/ml basic fibroblasts growth factor (R & D System, Abingdon, UK), 10 µg/ml insulin (Sigma)] at a density of 10,000 cells/ml. After 7 - 10 days culture, the mammospheres were photographed and subjected to further treatments.

**Flow cytometric analysis of CD24 and CD44 expression.** The adherent or mammosphere cells were trypsinised and passed through a 25G needle. The cells (2.5 × 10⁵) were incubated with CD24 and CD44 antibodies (BD Pharmingen, Oxford, UK) for 20 min at 4°C. Unbound antibodies were washed off with 2% FCS HBSS (Sigma) and the cells (10,000 events) were examined no longer than 1 hour after staining on a BD Facscalibur.

**The effect of different drug combinations on human breast tumour xenograft and mouse sarcoma in mice.** Five-week-old female athymic CD1 nude mice used in this study were housed under pathogen-free conditions. Human MDA-MB-231 breast cancer or mouse S180 sarcoma cells (1 × 10⁶) were injected s.c. at one flank of the mice. Tumour volume (V) was determined by the equation: V = (L × W²) × 0.5, where L is the length and W is the width of the tumour. When xenografts reached volumes of ∼200 mm³, the mice showing tumours were randomly grouped for the following treatments. The first day of drug treatment was designated as day 1.

Five groups (5 mice/group) were set up for mice with S180 sarcoma: 1. control, No treatment; 2. Lipo-DS, Lipo-DS 30mg/kg i.v.; 3. Lipo-DS/Cu, Lipo-DS 30mg/kg i.v. and CuCl₂ 3mg/kg oral; 4. DS, non-encapsulated DS 30mg/kg dissolved in vegetable oil, oral administration; 5. DS/Cu: DS 30mg/kg oral and CuCl₂ 3mg/kg oral.

Two groups (6 mice/group) were set up for human MDA-MB-231 xenografts treatment: 1. control, no treatment; 2. Lipo-DS/Cu, Lipo-DS 30mg/kg i.v. and CuCl₂ 3mg/kg oral.

The animals were subjected to different treatments when their tumours reached 2 mm in diameter. The drugs were administrated on Monday, Wednesday and Friday for two successive weeks. DS or Lipo-DS were administered in the afternoon. For the combination treatment groups, CuCl₂ were administered in the morning. The animals were observed for 21 (S180) and 28 (MDA-MB-231) days respectively.

**Statistical Analysis.** The data analysis was performed using T-test and One Way ANOVA.

## Claims

1. A disulfiram formulation comprising disulfiram, diethyldithiocarbamic acid or diethyldithiocarbamate (DDC) together with a component that increases the *in vivo* half life of the disulfiram, diethyldithiocarbamic acid or diethyldithiocarbamate (DDC) for use in the treatment of cancer, wherein the component that increases the half life of the disulfiram, diethyldithiocarbamic acid or diethyldithiocarbamate (DDC) is a liposome or a nanoparticle and wherein the disulfiram formulation is formulated for simultaneous or sequential administration with a separate copper containing formulation.

2. The formulation for use according to claim 1 wherein the disulfiram, diethyldithiocarbamic acid or diethyldithiocarbamate (DDC) is encapsulated in a liposome.

3. The formulation for use according to any one of the preceding claims wherein the disulfiram formulation does not comprise copper.

4. The formulation for use according to claim 1, wherein disulfiram, diethyldithiocarbamic acid or diethyldithiocarbamate (DDC) is the only active ingredient encapsulated.

5. The formulation for use according to any one of the preceding claims, wherein the *in vivo* half-life of disulfiram, diethyldithiocarbamic acid or diethyldithiocarbamate (DDC) is increased to be at least 30 minutes.

6. A pharmaceutical composition comprising a disulfiram formulation for use according to any one of the preceding claims and a pharmaceutically acceptable carrier or excipient.

## Patentansprüche

1. Disulfiramformulierung, umfassend Disulfiram, Diethyldithiocarbamidsäure oder Diethyldithiocarbamat (DDC) zusammen mit einer Komponente, die die In-vivo-Halbwertszeit des Disulfirams, der Diethyldithiocarbamidsäure bzw. des Diethyldithiocarbamats (DDC) erhöht, zur Verwendung bei der Behandlung von Krebs, wobei es sich bei der Komponente, die die Halbwertszeit des Disulfirams, der Diethyldithiocarbamidsäure bzw. des Diethyldithiocarbamats (DDC) erhöht, um ein Liposom oder ein Nanopartikel handelt, und wobei die Disulfiramformulierung für die gleichzeitige oder aufeinanderfolgende Verabreichung mit einer getrennten kupferhaltigen Formulierung formuliert ist.

2. Formulierung zur Verwendung nach Anspruch 1, wobei das Disulfiram, die Diethyldithiocarbamidsäure bzw. das Diethyldithiocarbamat (DDC) in einem Liposom verkapselt ist.

3. Formulierung zur Verwendung nach einem der vorhergehenden Ansprüche, wobei die Disulfiramformulierung kein Kupfer umfasst.

4. Formulierung zur Verwendung nach Anspruch 1, wobei es sich bei dem Disulfiram, der Diethyldithiocarbamidsäure bzw. dem Diethyldithiocarbamat (DDC) um den einzigen verkapselten Wirkstoff handelt.

5. Formulierung zur Verwendung nach einem der vorhergehenden Ansprüche, wobei die In-vivo-Halbwertszeit des Disulfirams, der Diethyldithiocarbamidsäure bzw. des Diethyldithiocarbamats (DDC) um mindestens 30 Minuten erhöht wird.

6. Pharmazeutische Zusammensetzung, umfassend eine Disulfiramformulierung zur Verwendung nach einem der vorhergehenden Ansprüche und einen pharmazeutisch unbedenklichen Träger oder Exzipienten.

## Revendications

1. Formulation de disulfirame comprenant du disulfirame, de l'acide diéthyldithiocarbamique ou du diéthyldithiocarbamate (DDC) conjointement avec un composant augmentant la demi-vie *in vivo* du disulfirame, de l'acide diéthyldithiocarbamique ou du diéthyldithiocarbamate (DDC) pour une utilisation dans le traitement d'un cancer, le composant augmentant la demi-vie *in vivo* du disulfirame, de l'acide diéthyldithiocarbamique ou du diéthyldithiocarbamate (DDC) étant un liposome ou une nanoparticule et la formulation de disulfirame étant formulée pour une administration simultanée ou séquentielle avec une formulation distincte contenant du cuivre.

2. Formulation pour une utilisation selon la revendication 1, le disulfirame, l'acide diéthyldithiocarbamique ou le diéthyldithiocarbamate (DDC) étant encapsulé dans un liposome.

3. Formulation pour une utilisation selon l'une quelconque des revendications précédentes, la formulation de disulfirame ne comprenant pas de cuivre.

4. Formulation pour une utilisation selon la revendication 1, le disulfirame, l'acide diéthyldithiocarbamique ou le diéthyldithiocarbamate (DDC) étant le seul principe actif encapsulé.

5. Formulation pour une utilisation selon l'une quelconque des revendications précédentes, la demi-vie *in vivo* du disulfirame, de l'acide diéthyldithiocarbamique ou du diéthyldithiocarbamate (DDC) étant augmentée pour être d'au moins 30 minutes.

6. Composition pharmaceutique comprenant une formulation de disulfirame pour une utilisation selon l'une quelconque des revendications précédentes et un vecteur ou un excipient pharmaceutiquement acceptable.
